# EUROPEAN PATENT APPLICATION

(11) **EP 1 652 921 A1**
(43) Date of publication of application: **03.05.2006**
(21) Application number: 04722033.0
(22) Date of filing: 19.03.2004
(51) Int. Cl.: C12N 15/11, C12N 5/14, A01H 1/00, A01H 5/00

(54) **RICE TRANSPOSON GENE**

(30) Priority: 04.07.2003 JP 2003270879
(71) Applicant: Japan Science and Technology Corporation, Kawaguchi-shi, Saitama 332-0012 (JP)
(72) Inventor: IIDA, Shigeru, 3-21, Tatsumigaoka-koumuin-shukusha, Okazaki-shi, Aichi 444-0874 (JP); MAEKAWA, Masahiko, Okayama-shi, Okayama 703-8275 (JP); TSUGANE, Kazuo, Okazaki-shi, Aichi 444-0877 (JP)
(74) Representative: Wilson Gunn
(86) International application number: PCT/JP2004/003772
(87) International publication number: WO 2005/003349

(57) **Abstract**

The present inventors identified the causative gene of pyl mutant, which is mutable and shows a lowered chlorophyll accumulation, and discovered that a new type of transposon classified to Ac/Ds type is involved in the mutation. It is confirmed for the first time that the Ac/Ds type transposon nDart (SEQ ID NO: 1) in rice has transposon activity under normal cultivation conditions. Furthermore, an autonomous element Dart was discovered by analyzing said Ac/Ds type transposon.

## Description

### Field of the Invention

The present invention relates to a rice transposon gene, more specifically to a rice Ac/Ds type transposon gene and it's autonomous element.

### Prior Art

Based on the type of the transposition, transposable elements are broadly classified into class I element, where a transposable element transposes via an RNA intermediate, and class II element, where a transposable element transposes as DNA molecule after cut out of DNA. A large scale rice gene tagging system has been developed using Tos17, a class I element, however, it has been known that highly frequent somatic mutations are induced, because Tos17 transposition accomplishes via a callus culture (Trend. Plant Sci. 6: 127-134 (2001)). Whilst, MITE-based mPing is reported as a class II element (Nature, vol. 421, No. 6919, pp.167-170 (Jan. 2003))

### Problems which this Invention Attempts to Solve

However, mPing transposition needs anther culture or γ-irradiation, which induces somatic mutation or highly frequent mutation (Nature, vol. 421, No. 6919, pp167-170 (Jan. 2003)). Moreover, it is considered that only a part of possible mutations is obtainable because of the target sequence specificity of these transposons. Therefore, a new tagging system is required.

### Means for Solving the Above Problems

The present inventors identified the causative gene of pyl (pale-yellow leaf) mutation, which is mutable and lowers the chlorophyll accumulation, and found that a new transposon, classified to a Ac/Ds type element, is involved in the mutation. These results confirmed for the first time the presence of Ac/Ds type transposon nDart (nonautonomous Ds-related active rice transposon), which has a transposon activity under the normal cultivation conditions of rice. Furthermore, the inventors discovered the autonomous element Dart during the analysis of the Ac/Ds type transposon.

Therefore, the present invention is a rice transposon gene (nDart) comprising DNA of the following (1) or (2);
(1) DNA comprising the nucleotide sequence of SEQ ID NO: 1.
(2) DNA comprising the nucleotide sequence, which is more than 98%
   homologous to the nucleotide sequence of (1), wherein said DNA transposes by subjecting rice containing said DNA to the treatment with a chemical agent.
   Furthermore, the present invention is a rice transposon gene (Dart) comprising DNA of the following (3) or (4);
(3) DNA comprising any nucleotide sequence of SEQ ID NOs: 6-8.
(4) DNA comprising the nucleotide sequence, which is more than 98%
   homologous to the nucleotide sequence of (3), wherein said DNA transposes by subjecting rice containing said DNA to the treatment with a chemical agent.

It has been known that the transposition frequency of a transposon increased by subjecting a plant to culture under the condition to induce active cell division. Therefore, for the increase of the transposition frequency of nDart and Dart, which transpose under normal cultivation conditions, furthermore, it is effective for plants to subject to grow under stressful conditions. The stressful conditions include the treatment with a chemical agent, 5-azacytidine which induce DNA demethylation, the exposure to radiation, such as UV and γ-rays, or the application of an artificial culture system, such as culture of callus, which is obtained by dedifferentiation of plant cells. Said treatments increase the transposition frequency of nDart and Dart and an increase in the mutation frequency enables to obtain efficiently the desired mutants.

Moreover, the present invention is a plasmid containing any transposon gene above described. The useful plasmids here include Ti plasmid and binary vectors such as pBI-121 plasmid etc. The useful promoters here include 35S promoter of cauliflower mosaic virus, heat shock promoter, chemotaxis promoter and others. In addition, there are no restrictions on the method of ligation between promoters and said gene, and general method of genetic engineering could be applied arbitrarily.

Also, the present invention is a transformant, wherein any one of said transposon gene is transduced. The host is preferably a plant and preferably Arabidopsis, tobacco, tomato, petunia, crucifer, cotton plant or maize. To transform the plant, using a general method of genetic engineering, these genes can be inserted into said plasmid and the plant can be transformed.

Also, the present invention is a transformed plants or seed, wherein any one of said transposon gene is transduced by any one of said methods. The plant is preferably Arabidopsis, tobacco, tomato, petunia, crucifer, cotton plant or maize.

### Brief Description of the Drawings

Figure 1 shows a mutable pyl-v mutant.
Figure 2 shows pyl-stb (left), whose phenotype is stable and wild type "Taichung No.65" (right). An individual piece at 7 days after seeding on modified white media is shown.
Figure 3 shows ply gene map-based cloning.
Figure 4 shows the result of a gel electrophoresis of PCR product of Example 1.
Figure 5 shows OsClpP gene (a ply mutant). A black square shows an exon and atg shows the initiation codon.
Figure 6 shows the flanking regions of the transcription initiation site of OsClpP gene in a ply-stb mutant. The small letters show the region to be translated to a protein and atg with an underline shows the initiation codon. The first arrow show the transcription initiation site of a wild type and other arrows show transcription initiation sites of ply-stb mutants. The numbers on a shoulder show the numbers of cloning.
Figure 7 shows the structure (A) of OsClpP gene of a revertant and the footprint (B) remained. The atg with an underline shows an initiation codon.
Figure 8 shows an autonomous element searched by BLAST.
Figure 9 shows the result of a gel electrophoresis of DNA of a mutated region of intercrosses (B3F1) between *indica* rice (Kasarasu) and pyl-v mutants of Taichung No. 65 (T-65). (1) shows the nDart1-0 (chromosome No.3) mutation , (2) nDart1-4(3-1) (chromosome No.3), (3) nDart1-12 (chromosome No. 1), (4) nDart200-2 (chromosome No. 12). Line 1 shows Kasarasu, Line 2 shows T-65, pyl-v and Line 3-21 shows intercrosses (B3F1).

### Detailed Description of the Invention

The inventors searched transposons with highly homologous to nDart using BLAST (Basic Local Alignment Search Tool). The database sites of the search are US Biotechnology information center and DNA data bank of National Institute of Genetics. The Query was nDart (SEQ ID NO: 1). RiceGAAS system (Yu.J, Hu.S, (2002) Science 296: 79-92) was used as a gene prediction program. The result of our analysis using BLAST is shown in Table 1. Thirty-one rice nucleotide sequences are homologous to nDart and 7 out of them are more than 98% homologous to nDart.

Any cultivar could carry an active DNA transposon as an efficient mutagen by crossing without an exogeneous gene for breed improvement, since the nucleotide sequence (SQE ID NO: 1) of nDart has been determined in the present invention.

By the transposition of nDart it is possible to obtain a mutant with disrupted gene and to isolate a stable mutant, wherein nDart re-transposed and a mutated footprint remained, and a revertant, wherein a mutated phenotype reverted to a wild one, from self-fertilized progenies of the mutants. It is possible to identify the gene disrupted by re-insertion of transposed nDart, by way of designing primers inside nDart sequence, performing reverse PCR and others. By analyzing the relation between these mutants and their genes, the function of the genes can be understood. In this case, the gene disrupted by a transposed nDart can be identified using said method.

Furthermore, it is possible to obtain a mutant with disrupted gene by transduction of the transposon to other plants by general transformation method. By analyzing the relation between these mutants and their genes, the function of the genes can be investigated. In this case, the gene disrupted by the transposed transposon can be identified using said method.

As a way to utilize nDart and Dart of the present invention, a tagging system with a transposon for rice or other desired plants can be produced by using these transposons as mutagens. In the case of producing a tagging system for rice particularly, an intercross easily enables any rice cultivar to carry nDart and active Dart genes. Since said tagging system dose not contain an exogeneous gene, it is possible to develop the tagging system in a large scale on an outside field, using a general cultivation method without using a physical containment facility, which is necessary for the cultivation of a genetically modified plant. The mutants thus obtained could be analyzed by a method of genetic or reverse genetic analysis. The genetic method is a method to isolate a causative gene based on the phenotype of a mutant and enables to identify and to isolate the causative gene easily using a tag (a transposon), since the transposon of the present invention and the phenotype of a mutant are linked.

Also, the reverse genetic analysis is a method of isolating mutants, wherein a certain function of a gene is lost out of the original genes. Firstly, DNA pool is prepared after isolation of DNA from various kinds of mutants. The pool was PCR screened and the screening enables to pick up a mutant, wherein a transposon is inserted to the expected gene.

### Examples

The following Examples illustrate this invention, however, it is not intended to limit the scope of the invention.

### Test Example 1.

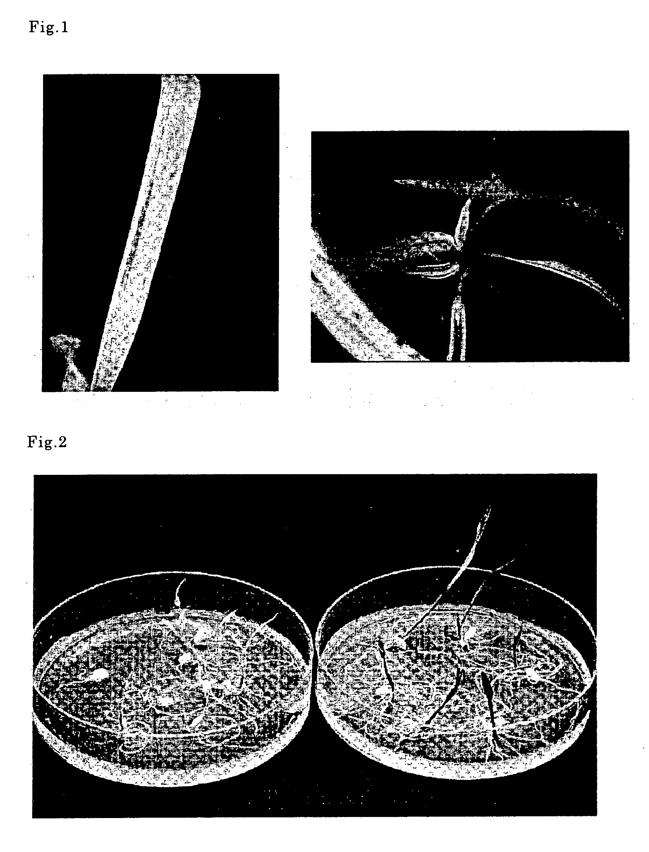

Maekawa, a co-inventor of the present invention, isolated a mutable pyl-v mutant (Fig. 1), which is generated by intercross (F2) between *japonica* rice H-126 and *indica* rice C-5052 and has a variegation with a lowered chlorophyll accumulation in 1986 (Fig. 1). Mutability is expected to be due to the insertion and release of a DNA-type transposon based on the observation that, in pyl-v mutants, a dark green-colored sector, similar to wild type, appears along with a cell group in a corn-colored leaf, as well as on the genetic analysis.

### Test Example 2.

Then, pyl-stb (pale-yellow leaf-stable) is isolated, wherein the pyl phenotype is apparently stable, after preparing a near-isogenic lines by repeated intercross between a pyl-v mutant obtained in Test Example 1 and *japonica* rice "Shiokari" or "Taichung No. 65".

Since pyl-stb (pale-yellow leaf-stable) leads to isolation again of a mutable line by an intercross and turns into a mutable line by the treatment with a chemical agent, pyl-stb line is transiently stable owing to separation or inactivation of an autonomous element.

A pyl-stb mutant seedling germinated on the soil is blighted before the fourth leaf stage. In this Example, pyl-stb seed was cultured on an agar media under sterile condition until the fourth leaf stage and then transplanted on the soil. In this way, pyl-stb resulted in fruitage with high probability. A pyl-stb seed can grow over the fourth leaf stage, if it is seeded and germinated under white fluorescent lamp with continuous light at 26 µmol m⁻² sec⁻¹, 28°C and sterile condition with modified white medium (Kusumi K., Mizutani A. et al. (1997) Plant J. 12: 1241-50). The pyl-stb grown over the fourth leaf stage can come to fruition with high probability after transplanted to the soil. Figure 2 shows the growth of pyl-stb seedlings.

### Example 1

In this Example, a map-based cloning was conducted in order to identify a DNA-type transposon gene, which induce variegation of pyl-v, and the causative gene of pyl mutation.

For the analysis of a map-based cloning, F2 population was used, which is generated by intercross between *indica* rice "Kasarasu" and near-isogenic lines, which are grown after backcross between pyl-v mutant obtained in Example 1 and "Shiokari". DNA were isolated from 21 pyl-v mutants which were derived from this F2 seedlings and a simple mapping of pyl was conducted by using 54 landmark SSR marker (Theor. Appl. Genet. 100:697-712 (2000)) covering 12 chromosomes. As the result, it was found that pyl gene is located within 22cM between RM282 and RM251 of the chromosome No.3 short arm.

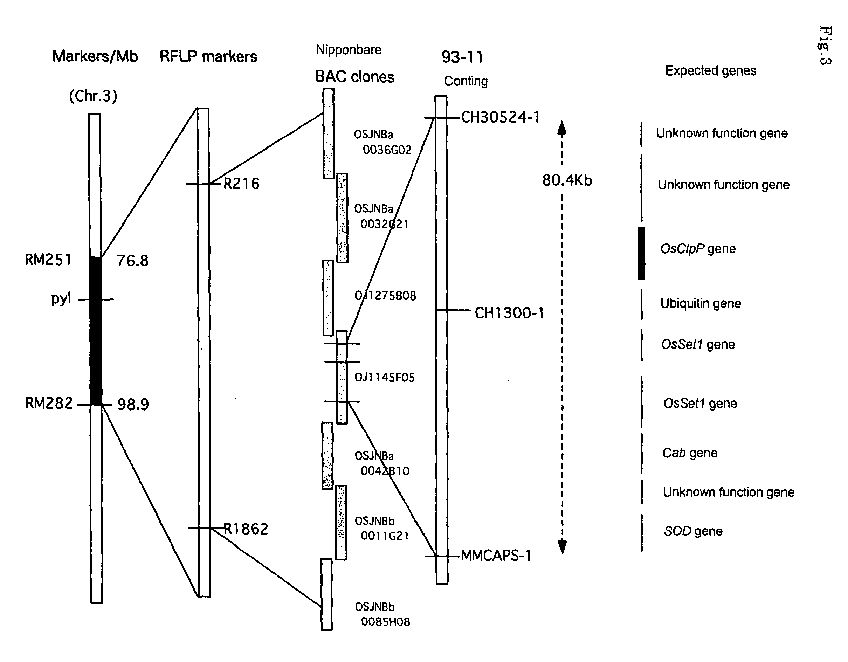

Then, EST clone (Plant cell 14, 525-35 (2002)) of Nipponbare located between the above 2 markers was selected. Based on the nucleotide sequence of these EST clones, a conting (Science 296: 79-92 (2002)) was selected, which is a group of linked genome DNA sequence of indica-type 93-11 containing these EST clones. At the same time, BAC clone of Nipponbare, reported by CSHL group in USA, was selected and 18 markers were prepared, which are usable as primers to get PCR product for identification, located straddling the DNA region, where there are more than 8 bp difference between the nucleotide sequences of the two selected clones. Using these markers, those seedlings expressing pyl phenotype out of 11800 F2 seedlings were selected and 3112 genetically modified seedlings were obtained. As the result, the candidate genes of pyl within about 80.4kb were obtained. Figure 3 shows the mapping diagram.

9 ORF were expected in this region, designed primer sets to amplify all these ORF regions of the genome and examined the PCR products of "Taichung No. 65", pyl-stb and "Kasarasu".

PCR reaction mixture (50 µl) contains 2.5U LA Taq, 1xGC buffer, 400 µM dATP, 400 µM dGTP, 400 µM dCTP, 400 µM dTTP (Takara), 0.2 µM primer set, 100 ng genomic DNA of "Taichung No. 65", pyl-stb and "Kasarasu", and sterilized MilliQ water (Milipore) for the volume adjustment. The PCR products were separated by 0.8 % LOIII agarose gel (Takara) electrophoresis.

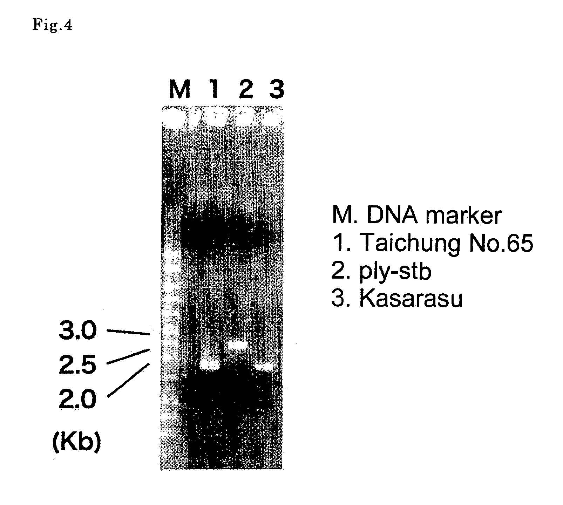

The PCR, with primers Clp-3F (SEQ ID NO: 2) and Clp-4R (SEQ ID: 3), resulted in about 600 bp difference between the products of pyl-stb and other lines. Figure 4 shows the result of the electrophoresis.

The gene amplified by the primers Clp-3F and Clp-4R is a gene (OsClpP, SEQ ID No: 9), which is 80% homologous to ClpP5 gene (Trend. Plant Sci. 6:127-134 (2001) of Arabidopsis and is probably coding a protease transported to chloroplast.

### Example 2

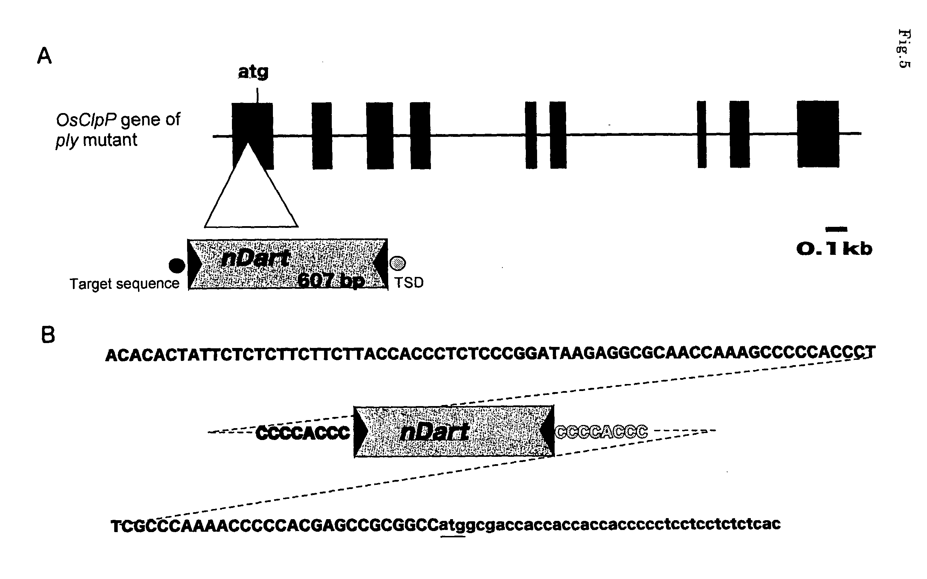

In this Example, the inventors determined nucleotide sequence of PCR products to confirm the difference of PCR products amplified in Example 1.
the PCR products of Example 1 were purified by QIA quick PCR purification Kit (Qiagen), determined the nucleotide sequences by the sequencer (ABI PRISM377, Applied Biosystem) and analyzed the sequences by various softwares. Then, it was found that a 607 bp sequence (SEQ ID NO: 1) is inserted to the exon 1 of OsClpP gene. Figure 5 shows the structure of the insertion site.

Eight bp TSD (Target Site Duplication) of the target sequence is induced at the time of DNA transposon insertion in this region and 19 bp TIR (Terminal Inverted Repeat) is generated at both ends of the 607 bp insert.

This insert sequence is classified to Ac/Ds type based on 8bp TSD and the similarity of TIR to known DNA transposon. Table 2 shows the comparison between the known Ac/Ds type transposon and this insert sequence (nDart). This insert sequence is a new type transposon gene similar to Ds without accompaniment of an autonomous element.

### Example 3

To determine the transcription initiation site and whole length of the gene, which is expected to code OsClpP protein and contains insert nDart (SEQ ID NO: 1) in a pyl mutant, in this Example, the inventors performed 5'RACE and 3'RACE to notice CAP structure of mRNA.

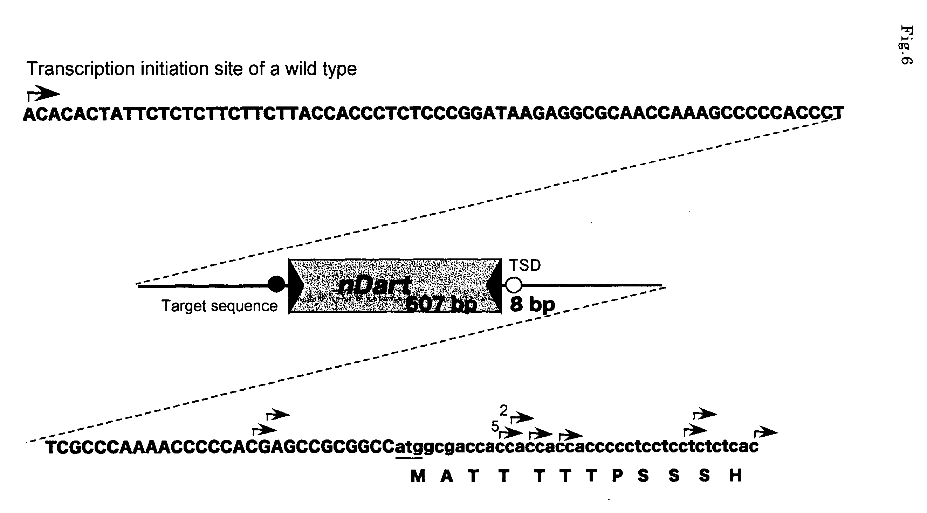

RNA was isolated from pyl-stb using guanidinium thiocyanate HCl and prepared cDNA from 1 µg total RNA using THERMOSCRIPT (Invitrogen). The transcription initiation site was determined by repeated PCR using the primer PG8-813R (SEQ ID NO: 4) and the primer Clp-3R (SEQ ID NO: 5), prepared on the basis of GeneRacer Kit (Iinvitrogen) and the nucleotide sequence of OsClpP gene, and using said cDNA as a template. Figure 6 shows the result.

It was found that most of the transcription initiation sites are located at the downstream of the sequence coding methionine, which is the first amino acid in the translated protein, in pyl-stb and thought that pyl mutation is due to OsClpP gene.

### Example 4

To examine the release of nDart in 49 revertants derived from 15 independent lines appeared from pyl-v mutants, in this Example, the inventors performed PCR using primers Clp-3F (SEQ ID NO: 3) and Clp-4R (SEQ ID NO: 4) to amplify the region from the first exon to the seventh exon of OsClpP gene.

PCR reaction mixture (50 µl) contains 2.5U LA Taq, 1xGC buffer, 400 µM dATP, 400 µM dGTP, 400 µM dCTP, 400 µM dTTP (Takara), 0.2 µM primer set, 100 ng genomic DNA of a revertant, and sterilized MilliQ water (Milipore) for the volume adjustment. The PCR products was separated by 0.8 % LOIII agarose gel (Takara) electrophoresis and confirmed that PCR products with the same size to that of the DNA fragment, wherein the transposon gene (SEQ ID NO: 1) is deleted, was amplified.

### Example 5

To confirm that the PCR products amplified in Example 4 was the result of transposition of the transposon gene (SEQ ID NO: 1) from the first intron region of OsClpP gene, in this Example, the inventors determined the nucleotide sequence of PCR products.

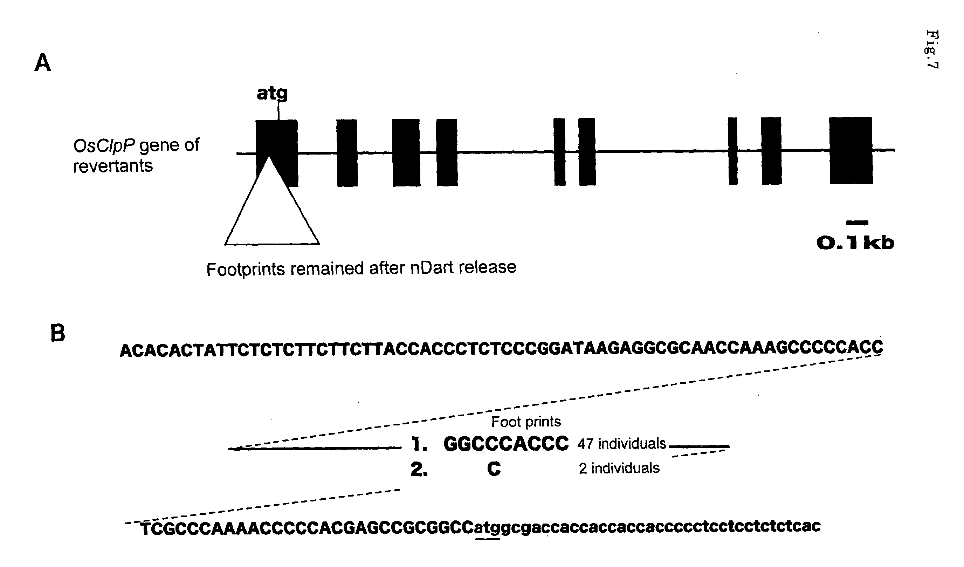

The PCR products of Example 4 were purified by QIA quick PCR purification Kit (Qiagen), determined the nucleotide sequences by the sequencer (ABI PRISM377, Applied Biosystem) and analyzed the sequence using various softwares. Figure 7 shows the results.

Any change on the exon 1 of OsClpP gene was not observed except the change of the nucleotide sequence by two kinds of footprint in said revertants. Also, the change by said footprint does not influence on the translation from mRNA of OsClpP to a protein and the same CLP protein to wild type might be produced in said revertants.

### Example 6

In this Example, it was confirmed that pyl-stb seeds obtained in Test Example 2 were able to be changed to a pyl-v mutant by the treatment with azacytidine treatment.

pyl-stb seeds were macerated in 0.15, 0.3 or 0.45 mM azacytidine aqueous solution for 24 hrs at 30°C, washed them, left for germination and examined the expression of pyl-v Table 3 shows the result. The frequency of the transposition of nDart increased as the concentration of azacytidine increased.

### Example 7

In this Example, autonomous element transposases controlling the transposition of nDart was searched by Blast (Basic Local Alignment Search Tool).

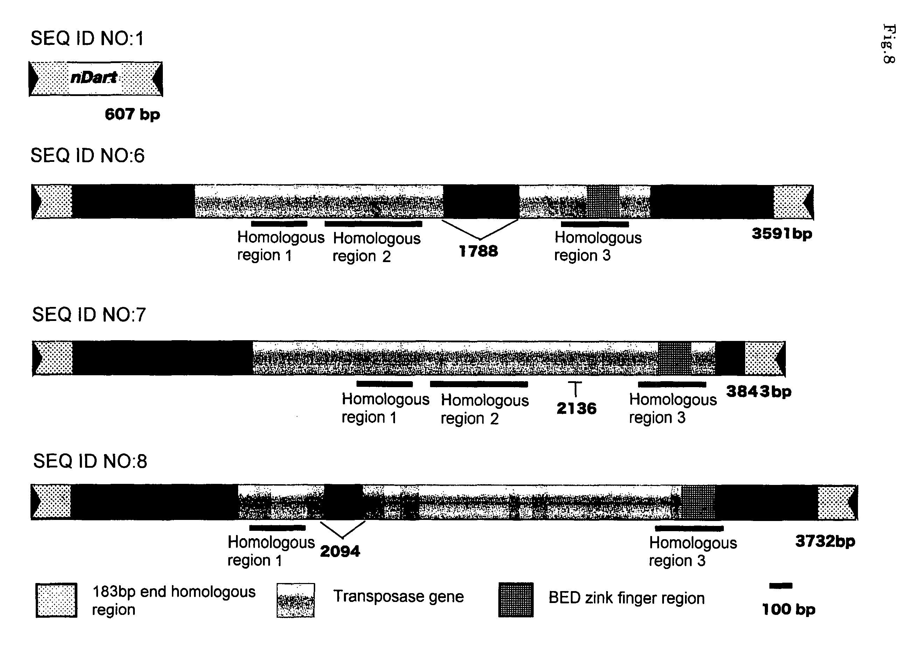

Since it was expected that a nucleotide sequence containing a transposase gene has the same sequence to that of nDart at both ends of the sequence and has transposase gene inside the sequence, the sequence having the same sequence of both ends of nDart (SEQ ID NO: 1) was searched and 3 sequences were found (SEQ ID NOs: 6-8). Figure 8 shows the sequences.

The sequence of SEQ ID NO: 6 has the end sequence with the highest homology among them. Each 183 bp of the both ends of SEQ ID NO: 6 is more than 98% homologous to the both ends of SEQ ID NO: 1 and it is shown by a gene prediction program that the ORF inside the sequence of SEQ ID NO: 6 has transposase genes. It was thought that the sequence of SEQ ID NO: 6 was an autonomous element, which is highly homologous to Tam3 transposase reported for Antirrhinum.

Then, an autonomous element having the highest homology to Tam3 predicted from the sequence of SEQ ID NO: 6 was searched. Since the transposase gene of Tam3 is reported to have a structure without introns, transposase genes without introns were searched from said 31 nucleotide sequences and the sequence of SEQ ID NO: 7 was identified. Based on the analysis by a gene prediction program, the sequence of SEQ ID NO: 7 has a structure without introns and contains BED Zinc finger region, a DNA binding region, at the 3' end. The sequence of SEQ ID NO: 7 may be an autonomous element controlling the transposition of nDart.

Based on the transposase gene predicted from the sequence of SEQ ID NO: 7 as an index, nucleotide sequences with different splicing patterns were searched and the sequence of SEQ ID NO: 8 was identified. As shown in Fig. 8, the sequence of SEQ ID NO: 8 has homologous regions 1 and 3 but not 2 and a different expression pattern and function from that of SEQ ID NO: 7 is expected.

### Example 8

*Indica* rice (Kasarasu) and pyl-v mutants of Taichung No. 65 (T-65) were intercrossed, obtained in Test Example 2, and nDart and an autonomous element (Dart) were transduced into Kasarasu. The backcross was performed for 3 times, and theoretically, the frequency of transformation to Kasarasu genotype is expected to be 93.8%.

In the case of pyl mutant, the causative gene of the mutation is nDart1-Origin (referred to as nDart1-0) inserted to OsClpP gene. By BLAST analysis, it was found that *japonica* rice (Nipponbare) has at least 14 nDarts, which are more than 98% homologous to nDart1-0. The nDart was named as nDart1-1 to nDart1-12, based on the degree of homology to nDart1-0. Since two sets of nDart (nDart1-3 and nDartl-4) has the same nucleotide sequence but locates on different loci of rice genome, they are classified according to the chromosome number shown in parentheses.

The mutated region of intercross (B3F1) lines between *indaca* rice (Kasarasu) and pyl-v mutant of Taichung No. 65 (T-65) was amplified by PCR. In the PCR, the oligonucleotides of SEQ ID NOs: 10 (F) and 11(R) for nDart1-0 (chromosome No. 3), those of SEQ ID NOs: 12 (F) and 13 (R) for nDart1-4 (3-1) (chromosome No. 3), those of SEQ ID NOs:14 (F) and 15 (R) for nDart1-12 (chromosome No. 1), those of SEQ ID NOs: 16 (F) and 17 (R) for nDart200-2 (chromosome No. 12) were used as primers.

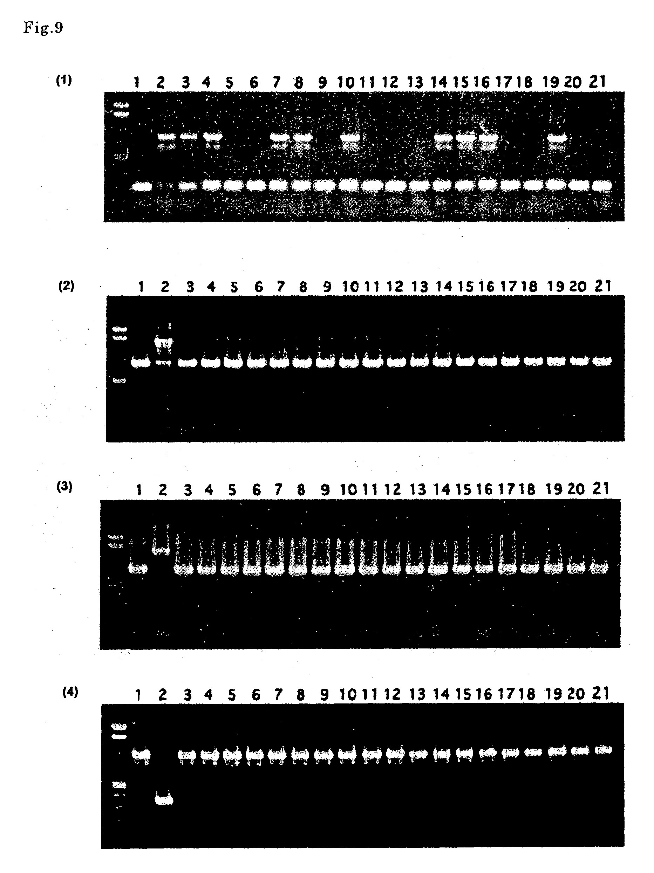

Figure 9 shows the result of gel electrophoresis of the mutated region of the intercross lines (B3F1, Fig.9, Line 3~12). nDart (SEQ ID NO: 1) was transduced to 9 out of 19 individuals (Fig 9 (1)). Since Kasarasu type bands are appeared at the same time, deletion of nDart is ongoing even in backcross individuals. Said nDart is located on the chromosome No. 3. Examining the extent of the transduction of Kasarasu on other chromosomes, it was found that the chromosome markers of chromosome No. 1, 3 and 12 are changed to Kasarasu type in all 19 individulas (Fig. 9, (2)-(4)). From the above results, it was concluded that nDart and an autonomous element (Dart) could keep their activity even in *indica* rice.

Furthermore, *indica* rice (93-11) was treated with azacytidine and confirmed release of nDart. Under usual growth conditions, release of nDart could not be observed, but the release was confirmed in 93-11 treated with azacytidine.

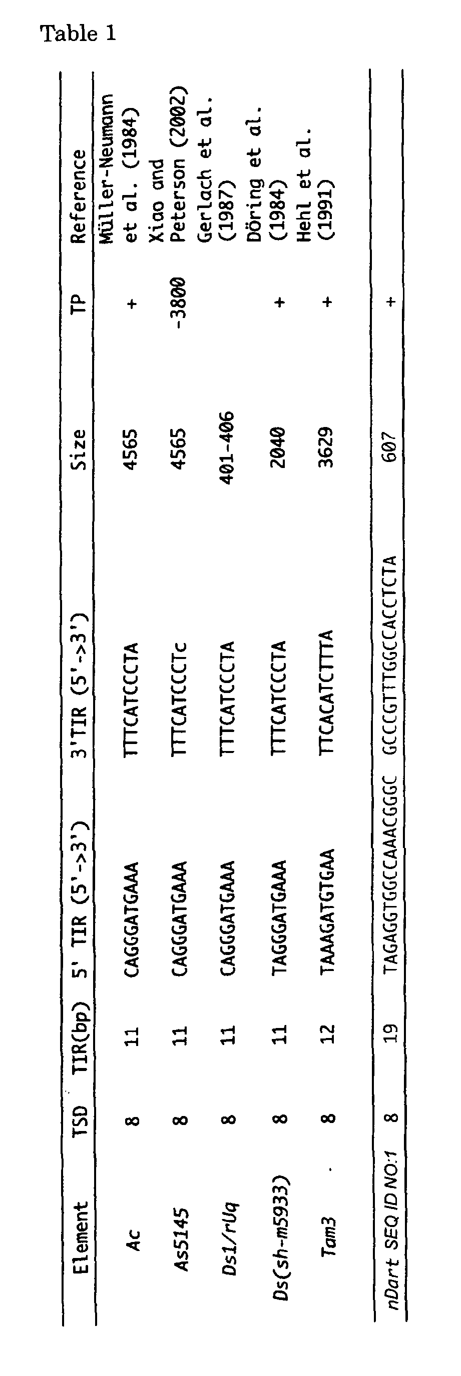

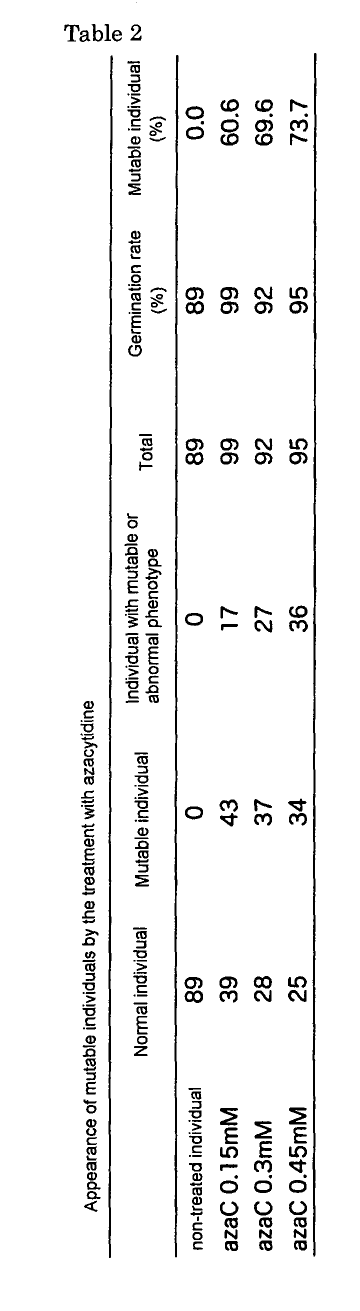

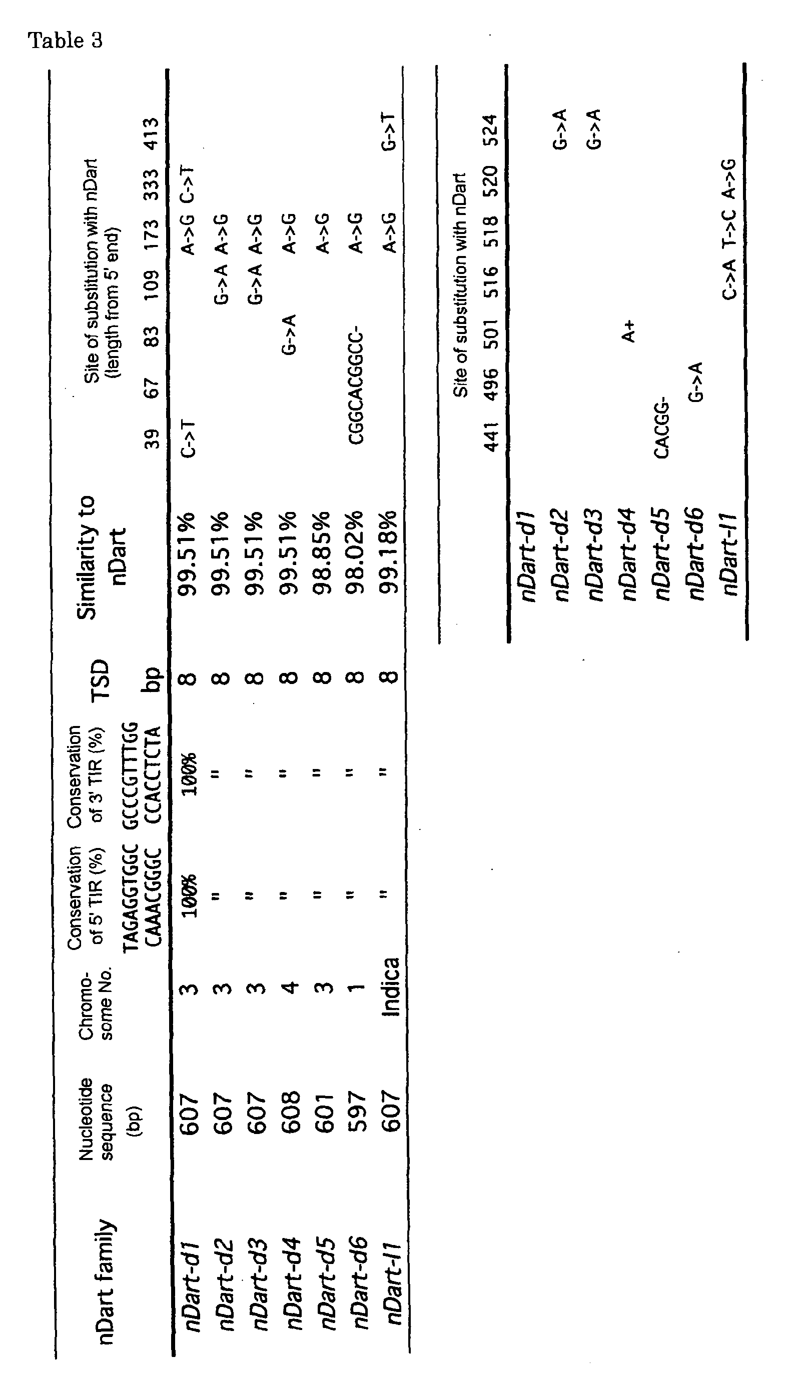

## Claims

1. A rice transposon gene comprising DNA of the following (1) or (2);
(1) DNA comprising the nucleotide sequence of SEQ ID NO: 1.
(2) DNA comprising the nucleotide sequence, which is more than 98% homologous to the nucleotide sequence of (1), wherein said DNA transposes by subjecting rice containing said DNA to the treatment with a chemical agent.

2. A rice transposon gene comprising DNA of the following (3) or (4);
(3) DNA comprising any nucleotide sequence of SEQ ID NO: 6 - 8.
(4) DNA comprising the nucleotide sequence, which is more than 98% homologous to the nucleotide sequence of (3), wherein said DNA transposes by subjecting rice containing said DNA to the treatment with a chemical agent.

3. The transposon gene as in claim 1 or 2, wherein said chemical agent is 5-azacytidine.

4. A plasmid comprising a transposon gene as in any one of claims 1 - 3.

5. A transformant transduced a transposon gene as in any one of claims of 1 - 3.

6. A transformant as in claim 5, wherein the host comprises a plant.

7. A transformant as in claim 6, wherein the host is arabidopsis, tobacco, tomato, petunia, crucifer, cotton plant or maize.

8. A method for transposing a transposon gene as in claim 1 or 2, which comprises treating a transformant as in any one of claims 5 - 7 with a chemical agent.

9. The method as in claim 7, wherein chemical agent is 5-azacytidine.

10. A transformed plant or seed, wherein said transposon gene is transposed by the method as in claim 8 or 9.


SEQUENCE LISTING

<110>  Japan Science and Technology Agency

<120>  Rice transposon gene

<130>  FS04-411PCT

<160>  17

<170>  PatentIn version 3.3

<210>  1
<211>  607
<212>  DNA
<213>  Oryza sativa

<400>  1
tagaggtggc caaacgggcc gggccaaaac gggcggcccg aggcacggcg gaacctgtag      60

ccggcacggc ccggcacggc ctgctacagt aacgggccgt gccggcacgg cacgagtagc     120

cgtgccgtgc ttgggccgcc ggccgagccc gcgggccggc acggcacggc acagctactg     180

tagtaagtcc gcatctcatc cttccgcaag tccgtatctc atccctccca actgacggcc     240

cagcccgcta gccgcctccg caagtccgtt gagcacccct cctagctgat ggcccagccc     300

gccagccacc tccgcaagtc cgcatcgcat ccctccgcgc catttcggtt cctgggccaa     360

ccgtgccccg tccacggccc atttttcatt cacgggcccg tactgacacg gcgggccaca     420

cgcatgccgt gccggcacgg gcacggcccg gccatccacg ggccgtgctt gggccggcgg     480

ctcggcacgt gggtcgggac ggcacggccc gtttcatgag ccgtgcctaa cgggccgtgc     540

cgaaacgggc cgtgccggac ccgtgcccgt gccgtgccgg gccgggccgc ccgtttggcc     600

acctcta                                                               607

<210>  2
<211>  20
<212>  DNA
<213>  Artificial Sequence

<220>
<223>  primer

<400>  2
taacgggtgt gtgtctggtg                                                  20

<210>  3
<211>  22
<212>  DNA
<213>  Artificial Sequence

<220>
<223>  primer

<400>  3
gcaactgaaa cccttacttg aa                                               22

<210>  4
<211>  20
<212>  DNA
<213>  Artificial Sequence

<220>
<223>  primer

<400>  4
gcggttgaag ggctttaagg                                                  20

<210> 5
<211> 20
<212> DNA
<213> Artificial Sequence

<220>
<223> primer

<400> 5
catcctccac gggtccacca 20


<210> 6
<211> 3591
<212> DNA
<213> Oryza sativa

<400> 6
tagaggtggc caaatgggcc gggccaaacg ggcggcccga ggcacggccg aacctgtagc 60
aggcacggcc cggcacggcc tgctacagta acgggccgtg ccggcacggc acgagtagcc 120
gtgccgtgct tgggccgctg gccgagcccg cgggccggca cggcacggca cggctactgt 180
agcgggccgg cacggcacgg cccgcggcac ggcacggcac ggcacgccgg cggcccgtca 240
ggcgcggaca gggcgggcgg cggtcgaatg ggaaggcgcc acgtggcact aacggctatt 300
tgaccgttca aatttgaaaa taaccgttgg gaggctaaaa aattcataaa aatttcgaaa 360
aaattccaaa aaatctcaaa tttcgcccta taaatagggc atgaacccca gccatttctc 420
ctcatcccac actcctcatc ttgtgctctc aagtgtttta agtgctctct ttgttctcaa 480
gtgtgcattt tttttgattt tgacaaaatt tgctcaaatt ttgtcaaaaa tcaaaattag 540
tttcgtagtt caacagtttg atcgcagagg tttgaagagc tcgcagttgg aaagatgtaa 600
gtaatattca aatttgtgta ttatttgtat tgtgtttgtg aattcaataa atattcgaaa 660
atttgtttat gtcggtttaa attttcagaa tggatccgaa ctttccatac cagtcgccgt 720
cgttcacctt gggtgatttc gaccccaact acatgtcggg gtttgatggt acctccggat 780
cggctccaac tccaccatct gtggaggagg taccggttca tacggctgtc gttgaggagg 840
taccggttca ggcggagaca gcttcggaag gattttccgg aaccgcgagc ggaagtgttt 900
cgacacacac cggctcgaag agatcgagaa cctccggtgt gtggcaaagc ttcgatgaga 960
taaaggaaac atgccccgac ggaagggagg tatcgaaagc ccgttgtaga atatgtaggc 1020
aaattttatc tgctcgttct tctggtggta caggtcacct caagcgccat gcggagtcgt 1080
gtgccaagaa gcaaggaata caactccggc agcagcaact tatggtaaac ccagacggta 1140
cggtacacag ttgggagtac gatcccatgg ttgctcggga atctcttgtc cggttaatcg 1200
ccaggcaaga tttacccctg aactttgggg agtcccctgc ttttgaacat tacattcagc 1260
aatctcataa ccctaggttt aaagctgtga gtaggcaaac atcaactaga gatttagaga 1320
atgtttatca caaggaagca actgcactta aggaactgtt tagtacatgt actttctctg 1380
ttagtgttac ttcagatata tggagtagta gagctagaga ggattatctt agcgtagttg 1440
ttcattttgt tgatgatgat tggcaattac aaaagagagt tttagggctt aggttaatag 1500
atgtctcaca tacaggagaa aacatagctg aaagaattag ggaagtaatt aatgaattta 1560
atcttgctga taaaatattt gctgtcaccc tagataatgc atctgctaat tctagggcta 1620
ttgaaatatt gcaaccttta ttttgtgtgt atgctcaatc ttttctactc catcagcgtt 1680

gtgcatgtca tataattaat ttgattgtta agactggcat gaagagggta ggtgaccaca 1740
tcgatgctgt tcgtcaagca atcgcgtggt taactgcttc taacccgcgg attgctgcat 1800
ggaagaggtt ttgcaatgcg gccggtgtga aagctcgtaa gtttgccacc gatgcagagc 1860
atcggtggaa tgcaacgtat ttaatgttaa aagttgtttt accttatagt agtttacttt 1920
ctgattttgt tcagtcacgt ggtggcccaa gaaacagtga cgggtcttca gtactgaacg 1980
agcatgtttg ggcaattgtc caaaaatttt accaatttct agaaactttt tatgattgta 2040
ctctaacttt gtcacaagtt tattatccaa ctgctaatat aattttgcac aaccttcttg 2100
aaattgctac tttatttaaa gaatacgaaa atgatgacgt tctaactgaa cctgtctttc 2160
acatgaaaca aaaatatttg aaatattgga aaaatatacc tatgttgtat gctcttgctt 2220
ttgttttaga tcctaggtgt aaattaaggg gattgtctgc tattttatca cttgttggag 2280
atactatagg tgtagattat agttcttttt atactgaggt tagacgtaaa ttatatgagg 2340
tttttggaag atatgaagta aagtttcagg aagttcgcca gcagagaccc cctcctatcc 2400
ccactacagg taagaagaag atacagtggg gtaggatttg gggtggatcg tcttcaagtt 2460
caatccaagg tggtggcagt tcgtcggcta caagtggaga cgcctcttcg catgttgtgg 2520
ccgaagagtt gtccggttat ttggacagcg acgccatcca ccacgaagca caagatttca 2580
acgtcctcgg gtggtggaat gaccacaaga taacatatcc tgtgctttca aaactagcac 2640
gggatgtgtt gacggtgccc gtgtcgacgg tgtcctccga atcggccttc agtctatgcg 2700
gccgaatcat cgaagaccgg aggacgactc tgcgcagcga ccacgtcgaa atgctactaa 2760
gcgttaaaga ctgggagctt gctcgacaac atgcccaata cactgcggac aaccaagaat 2820
tggctgccca gttcgagcaa ctctacctgg atccagacca accccagtag aattttgtta 2880
gaagtagttc tgacctttga gctgtactct tttctttgtc atggttttct cattttcccc 2940
tatgagtttt tacatgacaa agtttttaaa gaggcagcat gtatcattgt atcctgtaat 3000
gatataaaca tcaataaagg tcattactat ttttaacaaa ttcttttgca atattttcgc 3060
aagtgtggat ttatctttaa attatttcaa aataatgaat cacaatctat atttttaaat 3120
ttttcaacac aacaaaaaaa taccattttt tctttttttt aacattagca aatcattact 3180
ttttaaaaaa acttttattt ccatttttta aataccattt tttcattttt taacattagt 3240
aaatcattac ttttttttaa acattttatt tccattttta attttttttt tccttataca 3300
tttcctttgc ttttttttaa aaaaaaaaca ctgtgcacta caggctggcg ggctggcggc 3360
ctgccttcac gggccgccgt gccccgaacg gcccgtgggc cgcgggcgtg ccgtgccggc 3420
acgggcacgg cccggccatc cacgggccgt gcttgggccg gcggctcggc acgtgggccg 3480
gcacggcacg gcccgtttca tcagccgtgc ctaacgggcc gtgccgaaac gggccgtgcc 3540
ggaaccgtgc ccgtgccggg ccgggccgtg ccgcccgttt ggacacctat a 3591

<210> 7
<211> 3843
<212> DNA
<213> Oryza sativa

<400> 7
   tatacctggc caaatgggcc gtgccaagcc gggccggccc aagcacgacc gcactgtaga 60
   aggcccaggc ccggcacggc acgccggcct gtgggccgtg ccggcacggc ccgtttaccc 120
   gtgccgtgtc tgggccgacg ccatagcccg tgggccagca cggcacggca cgtttactgt 180
   agagggctgg cacggcccgg cacggcccgc gagcacggca cggcacggga tcggctcggc 240
   cgcgcacacc gcggcaccgc gcacgtggcg ccaagcggcc gagccgccga gggcagccgc 300
   ggggccaggc ggcgggaagc gcgcgtcgct tcgttcgcgc gtggcgcgtg gcaagcggcg 360
   tcgcgacgtg tcggcgtgtc gctggggctg gccagctggg aggctgggac gctgcctcac 420
   gctgggtcgg tcgctcactc gctctcgctg cctgtctgcc tgtctgccac tctgcctccg 480
   tgcctcgttg ggagcagccg agacagcgac tcggcgagac cccgtacggc ggccgaacgg 540
   ctagtcaaac gacgaatgcg agagtgccac gtgtccccca acggctagtg agctaatcca 600
   acgaccagaa gtagccgttg gagagcaaaa aaaaaggaaa aaaattcgaa aaaaatatga 660
   aatttatttc tataaatagg acacccacca gagcattctg aatcatccat acctcccatt 720
   ttgtgctctg ttgtgctctt tcgtgtgata gatcgatttt ttgatttaga caaaatttga 780
   caaaattttg tgtaaaatca aaaatagttt gtgactaaaa atagtgcata caagaggtgt 840
   aaagccaagc aaaggtggta agaaagaagt acgtcatata ttcagtttta tgtattattt 900
   tattttatgt tatgcgaata aatattctga aatttgttta tgttgtttta aattttcaga 960
   atggatgaat cgaacattcc atcgttcacg ttaggtgatt tcgaccctaa ctacgtgtcg 1020
   aggtcattcc caactggtga gtatgatgcc accggatcgg ctccaacacc accagttatg 1080
   gagccaccgg cgggttcaga agcatccggc actatgagtg ggagtgcatc gacgaacacc 1140
   ggctcaaaga gatcaagaac ttccggtgtt tggcaacatt tcgatgaggt ggccatgaca 1200
   ggccctgatg gaaggcaggt aacattcgcg agatgtagaa tatgcaaaaa taagttatct 1260
   gcaaaatcat ctggtggaac aggacatttg aagcggcatg ccgaggcttg tgcaaagaag 1320
   caaggaatcc aactacgaca gcaacaacta ctactaaatc ctgatggtac ggtacgtacg 1380
   tgggagtatg atcctatggt agctcgagaa aatcttgccc gtttaattgc tagacaagat 1440
   ttacccttga actttggtga gagtcctgca tttgaaaatt acataaaaaa ttctcataat 1500
   cctaggtttc aagctgttag tagacaaacc acaacccgtg atttgaaaaa tgtctatgac 1560
   aaaggttatg aatcactgaa ggaattattt agtacatgca ccttttctgt cagtgtcacc 1620
   tcagacatat ggagtagtag ggctaaagag gattacctta gtgtagttgt acatttcatt 1680
   gatgatgatt ggcaaatgca aaaaagagtt cttggcttaa ggttaattga tgtttcacat 1740
   actggtgaaa atatagcaga gagaattcga gaggttattg atgagtttaa ccttgcagat 1800
   aaaatttttg ctgtaacaat ggataatgca tctgcaaatt ctaggcccat ggaaattcta 1860
   caaccattat tttgtattta tgctcaatca tttcttctgc atcagcgttg tgcatgccat 1920
   atcattaatc taattgttaa atgtgggttt aagagagtta atgtacacat cgacgctgtt 1980
   cgtcaagcaa tcacgtggtt aactgcttca aacccacgga ttgcacagtg gaaaaggtat 2040
   tgttgtgcat cgggtgagcc cccacgtaag tttttaaccg atgcagacca tcggtggaat 2100
   gccacttatt ttatgttaaa ggttgtatta ccttacaagg atttacttac tgttttcctt 2160
   caaacacgta atggcccaaa aaacagtgat ggccagccaa tactgactga tcatacctgg 2220
   cacattgttg aaaggttcaa tcaatttctt gaaacgtttc atgactgtac tcttctgtta 2280
   tctcaagtat attatccaac agctaattta attttgcata atattcttga aattgccact 2340
   ttgttgaaag agtatgaaaa tgatgacctt ttaatgcccg ttgtctttaa tatgaaacaa 2400
   aaatatctta aatattggaa agacatcccc atgttgtatt cttttgcatt tattcttgat 2460
   cctaggggaa aattacgggg attcctcaat attctttcac ttattggaga tattattaat 2520
   gttgattatt ctacctatta tgctgatgtc aaaactaaat tctatgaggt atttcgaaag 2580
   tatgaattaa agtttcaggg agatcgcttg caaagacccc cacctgtacc tgcagcaggt 2640
   aagaaaaaat tacagtggag cagaatttgg ggcagttcat cttctagcca tggtggtggt 2700
   accagttcat cagcagcaag tggggacgct agatcgcatg gtcctgccga agagttgtcc 2760
   aactatttgg atagcgatgc catcaggcat gaaacgtcag acttcaacgt actcgggtgg 2820
   tggaatgatc ataagatgtc atatcctgtg ctatcaaaac tagcacggga tgtgttgacg 2880
   gtgcccgtat cttcggtatc ctccgaatca gccttcagtc tatgcggaag aattatcgag 2940
   gataggagaa caagtctgag cagcgatcat gtggaaatac tattaagcgt caaagactgg 3000
   gaacttgctg cagaacatgc ccaatacact gctgacaacc aagaattggc cgcacagttc 3060
   gaaaaccttt atttagatga cgaacaatta gggtagctag tttatatttt ttaagtattg 3120
   acctgttggc tgtactcttt tctttgtcat ggttttctca aatatgagtt tttacatgat 3180
   aaagttttta acgaggcagc atgtatcatg taaacatcaa taaaggtcat tactcttttt 3240
   tcctcatatt tttctaatat ttttctaagt ctaattattt ttctattttt ctccaactat 3300
   ccattaattt tctcttagct tagttaactt tcagaccttt ctctttgatt tgaattgttc 3360
   cactgacaga gtgacagcct gacagtgaca gactgacagg caatagacac acggtgacgg 3420
   acagcgtcag caagtccagc gccaccgccg ccacgtgtcg cccttcggcc ggccggtcgc 3480
   gcggccccgg ccgctcgctc ccgcgtgccg cgttgaaaat ttcagccgcg ccgcgcgcgc 3540
   gccttgtcgg cgactcggcg ttgtcgccta gccgagtcct tcggccgtgc cgcgtgcccg 3600
   cgtccttggc tgcagtccgt cgtgccaacg ggctgaccac ggcccatggg ccattgacgt 3660
   gcccgtgccg gcacggcacg gcacgacgtt ccctcgggcc gtgcttgggc cggggagtag 3720
   gcacgtgggc cggcacggca cggcccgcta taggagtcgt gcctaacggg ccgtgcccta 3780
   gcgggccgtg ccgccggcgt gcccgtgccg tgctgggccg ggccgcccgt ttggccaggt 3840
   ata 3843

<210> 8
<211> 3732
<212> DNA
<213> Oryza sativa

<400> 8
tatacctggc caaatgggcc gtgccaagcc gggccggccc aagcacgacc gcactgtaga 60
aggcccaggc ctggcacagc acgccggcct gtgggccgtg ccggcacggc ccgtttaccc 120
gtgccgtgtc tgggccgacg ccatagcccg tgggccagca cggcacggca cgtttactgt 180
agagggctgg cacggcatgg cacggcccgc gagcacggca cggcacggga gcggcctagg 240
gtaggcacac cgcacacgtg gcgccaagcg gccgagccgc cgagggcagc cgcggggcca 300
ggcggcggga agcgcgcgtc gctgcgttcg cgcgtggcgc gtggcaagcg gcgtcgcgac 360
gtgtcgctag ggctgggagg ctgggtcgct ctcgctctga ctgcctccgt cactccgtgc 420
ctcgttggga gcagccgaga cggcgacagg cgactcagcg agaccccata cggcggccga 480

acagctagtc aaacgacgaa tgcgagagtg ccacgtgtcc ccaacggcta gtgagctaat 540
ccaacgaccg ctgtttttga gaagtagccg ttggagagca aaaaaatgga aaaaaattcg 600
aaaaaaatat gaaatttatt tctataaata ggacacccac cggagcattc tgaatcatct 660
atacctccca ttttgtgctc tgttgtgctc tttcgtgtga tagatcgatt ttttgattta 720
gacaaaattt tgtctaaaat caaaaatagt ttgtgactaa aaatagtgca tacaagaggt 780
gtaaagccaa gcaaaggtgg taagaaagaa gtacgtcata tattcagttt tatgtattat 840
tttattttat gttatgcgaa taaatattct gaaatttgtt tatgttgttt taaattttca 900
gaatggacga atcgaacatt ccatcgttca cgttaggtga tttcgaccct aactacgtgt 960
cgaggtcatt cccaactggt gagtatgatg ccaccggatc ggctccaaca ccaccagtta 1020
tggagccacc agcgggttca gaagcatccg gcgctatgag tgggagtgca tcgacgaaca 1080
ccggctcaaa gagatcaaga acttccggtg tttggcaaca tttcgatgag gtggccgtga 1140
caggccctga tggaaggcag gtaacattcg cgagatgtag aatatgcaaa aataagttat 1200
ctgcaaaatc atctggtgga ataggacatt tgaagcggca tgccgaggct tgtgcaaaga 1260
agcaaggaat ccaactacga cagcaacaac tactactaaa tcctgatggt acggtacgta 1320
cgtgggagta tgatcctatg gtagctcgag aaaatcttgc ccgtttaatt gctagacaag 1380
atttaccctt gaactttggt gagagtcctg catttgaaaa ttacataaaa aaattctcat 1440
aatcctaggt ttcaagctgt tagtagacaa accacaaccc gtgatttgaa aaatgtctat 1500
gacaaaggtt atgaatcact gaaggaatta ttaagtacat gcaccttttc tgtcagtgtc 1560
acctcagaca tatggagtag tagggctaaa gaggattacc ttagtgtagt tgtacatttc 1620
attgatgatg attggcaaat gcaaaaaaga gttcttggct taaggttaat tgatgtttca 1680
catactggtg aaaatatagc agagagaatt cgagaggtta ttgatgagtt taaccttgca 1740
gataaaattt ttgctgtaac aatggataat gcatctgcaa attctagggc catggaaatt 1800
ctacaaccat tattttgtat ttatgctcaa tcatttcttc tgcatcagcg ttgtgcatgc 1860
catatcatta atctaattgt taaatgtggg tttaagagag ttaatgtaca gatcgacgct 1920
gttcgtcaag caatcacgtg gttaactgct tcaaacccac ggattgcaca gtggaaaagg 1980
tattgttgtg catcgggtga gcccccacgt aagtttttaa ccgatgcaga ccatcggtgg 2040
aatgccattt attttatgtt aaaggttgta ttaccttaca aggatttact tactgttttc 2100
cttcaaacat gtaatggccc aaaaaacagt gacggccagc caatactgac tgatcatacc 2160
tggcacattg ttgaaaggtt caatcaattt cttgaaacgt ttcatgactg tactcttctg 2220
ttatctcaag tatattatcc aacagctaat ttaattttgc ataatattct tgaaattgcc 2280
actttgttga aagagtatga aaatgatgac cttttaatgc ccgttgtctt taatatgaaa 2340
caaaaatatc ttaaatattg gaaagatatc ctcatgttgt attcttttgc atttattctt 2400
gatcctaggg gaaaattacg gggattcctc aatattcttt cacttattgg agatattatt 2460
aatgttgatt attctaccta ttatgctgat gtcaaaacta aattctatga ggtatttcga 2520
aagtatgaat taaagtttca gggagatcgc ttgcaaagac ccccacctgt ccttgcagca 2580
ggtaagaaaa aattacagtg gagcagaatt tggggcggtt catcttctag ccatggtggt 2640
ggtaccagtt catcagcagc aagtggagat gctagatcgc atggtcctgc cgaagagttg 2700
tccaactatt tggatagcga tgccatcagg catgaaacgt cagacttcaa cgtactcggg 2760

tggtggaatg atcataagat gtcatatcct gtgctatcaa aactagcacg ggatgtgttg 2820
acggtgcccg tatcttcggt atcctccgaa tcagccttca gtctatgcgg aagaattatc 2880
gaggatagga gaacaagtct gagcagcgat catgtggaaa tactattaag cgtcaaagac 2940
tgggaacttg ctgcagaaca tgcccaatac actgctgaca accaagaatt ggccgcacag 3000
ttcgaaaacc tttatttaga tgacgaacaa ttagggtagc tagtttatat tttttaagta 3060
ttgacctgtt ggctgtactc ttttctttgt catggttttc tcaaatatga gtttttacat 3120
gacaaagttt ttaacgaggc agcatgtatc atgtaaacat caataaaggt cattactctt 3180
ttttccccat atttttctaa tatttttcta agtctaatta tttttctatt tttctccaac 3240
tatccattaa ttttctctta gcttagttaa ctttcggacc tttctctttg atttgaattg 3300
ttccactgac agagtgacag gcgatagaca cacggacaga ggcaagtcac tgagtcagca 3360
ttcagcaagt ccagcgccac gtgtcgccct tcggccggcc ggtcccgcgg ccccggccgc 3420
tcgctcccgc gtgccgcgtc caaattttca tccgcgcgct cgccttgtcg gcgttgtcgc 3480
cttgccagct tgcctgcagt cgatcgtgcc aacgggccga ccacgaccca tgggccattg 3540
acgtgcccgt gcaggcacgg cacggcacga cgttccctcg ggccgtgctt gggccgggga 3600
gtaggcacgt gggccggcac ggcacggccc gctacaggag tcgtgcctaa cgggccgtgc 3660
cctagcgggc cgtgccgctg gcgtgcccgt gccgtgctgg gccgggccgg gccgcccgtt 3720
tggccaggta ta 3732

<210> 9
<211> 1186
<212> DNA
<213> Oryza sativa

<400> 9
acactattct ctcttcttct taccaccctc tcccggataa gaggcgcaac caaagccccc 60
accctcgccc aaaaccccca cgagccgcgg ccatggcgac caccaccacc accccctcct 120
cctctctcac cgcccctctc ctccgcccga gctcgaacgc gaaccccgcc ccgagatctc 180
tgccgctcct caggagccgg aggtgcgctc gggccgtggc gaccgccgcc gccgccgctg 240
gccacggggc cgctcatcag aggagcggga tttggtcgat cagggatgat ttggtggtgc 300
cgaggtcgcc ctacttccct gtggagtatg cgtcggggca ggaacgcggg ccatcgccca 360
tggtgatgga gcggttccag agcgtcgtca gccagctctt ccagcacagg attatccggt 420
gtggtggacc cgtggaggat gatatggcga acatcatcgt tgcccagctg ctatatctcg 480
atgccatcga tcctaacaag gatatcatta tgtatgtgaa ttctcctgga ggatcagtga 540
cagctgggat ggccatattc gacacgatga agcatatcag acctgatgtt tccacagttt 600
gtattggact tgctgcaagt atgggagctt ttctgcttag tgctgggaca aaaaggaagc 660
gatacagctt acctaactca agaataatga tccatcaacc tctcggagga gcccaaggac 720
aagagactga tcttgagatc caggctaatg agatgctgca tcacaaggct aacctgaatg 780
gatacctagc ataccacact gggcagcccc tagataagat caacgtagat actgaccgtg 840
attacttcat gagcgcgaag gaggcaaagg agtatggtct aattgatgga gttatcatga 900
atccccttaa agcccttcaa ccgcttcctg cttctagtta gccatggagt gctcaatctc 960
cacggagcat tttttggtta tcttttagaa ctgttattgc atccactgtt tttattagct 1020

tggcaagata gttttgcgat tccacaagca accacatcct gaggcttcaa agtttgtaca    1080

atacagatgt actactagga ggatatcttc tgcgatgaat attgcaactt atttgatgta    1140

ctattaggag gatatcttct gcgatgaata ttgcaactta tttgat                   1186

<210>  10
<211>  20
<212>  DNA
<213>  Artificial sequence

<220>
<223>  primer

<400>  10
gataagaggc gcaaccaaag                                                  20

<210>  11
<211>  20
<212>  DNA
<213>  Artificial sequence

<220>
<223>  primer

<400>  11
tggagccacg acagagtaga                                                  20

<210>  12
<211>  20
<212>  DNA
<213>  Artificial sequence

<220>
<223>  primer

<400>  12
gcactggtaa aatcgcctgt                                                  20

<210>  13
<211>  20
<212>  DNA
<213>  Artificial sequence

<220>
<223>  primer

<400>  13
aggtggattg cagccttatg                                                  20

<210>  14
<211>  20
<212>  DNA
<213>  Artificial sequence

<220>
<223>  primer

<400>  14
gaaacgcacc gtttagcaat                                                  20

<210>  15
<211>  20
<212>  DNA
<213>  Artificial sequence

<220>
<223>  primer

<400> 15
atttgagcac gaggaggaga 20

<210> 16
<211> 20
<212> DNA
<213> Artificial sequence

<220>
<223> primer

<400> 16
ttatctggga gggtgcagac 20

<210> 17
<211> 21
<212> DNA
<213> Artificial sequence

<220>
<223> primer

<400> 17
cgatttggtc tttccgttag a 21